# EUROPÄISCHE PATENTSCHRIFT

(11) **EP 2 983 866 B1**
(45) Veröffentlichungstag und Bekanntmachung des Hinweises auf die Patenterteilung: **25.01.2017**
(21) Anmeldenummer: 14716600.3
(22) Anmeldetag: 11.04.2014
(51) Int. Cl.: B24D 3/34, A45D 29/04, A61C 3/06, A61B 17/54, B24D 7/00, B24D 11/00

(54) **SCHLEIFKÖRPER**
GRINDING BODY
CORPS ABRASIF

(30) Priorität: 11.04.2013 DE 102013103643
(43) Veröffentlichungstag der Anmeldung: 17.02.2016
(73) Patentinhaber: Lukas-Erzett Vereinigte Schleif- und Fräswerkzeugfabriken GmbH & Co. KG, 51766 Engelskirchen (DE)
(72) Erfinder: RUNDEN, Bernhard, 53332 Bornheim (DE); FISCHER, Gerd, 51643 Gummersbach (DE)
(74) Vertreter: Neumann Müller Oberwalleney & Partner Patentanwälte
(86) Internationale Anmeldenummer: PCT/EP2014/057418
(87) Internationale Veröffentlichungsnummer: WO 2014/167111

(56) Entgegenhaltungen:
- DE-U1-202007 012 818
- DE-U1-202009 006 069
- US-A- 6 145 512
- US-A1- 2007 221 238

## Beschreibung

Die vorliegende Erfindung betrifft ein Schleifwerkzeug nach dem Oberbegriff des Anspruchs 1. Des Weiteren betrifft die vorliegende Erfindung eine Verwendung eines solchen Schleifwerkzeuges.

Schleifkörper und Schleifwerkzeuge dieser Art sind bekannt und werden zum Beispiel für die Metall- oder Holzbearbeitung, zur Herstellung von Prothesen oder für die Fußpflege und Maniküre eingesetzt. Da es bei Schleifvorgängen temporär und lokal zu einer hohen Wärmebelastung kommen kann, ist es immer wieder notwendig, den Schleifvorgang zu unterbrechen, um den Schleifkörper und die zu bearbeitenden Bereiche abzukühlen. Des Weiteren ist bekannt, den Schleifkörper während des Schleifvorgangs zu kühlen, um wärmebedingte Schmerzen beim Patienten oder Hitzeschäden an einem zu bearbeitenden Werkstück zu verhindern.

Ein Schleifwerkzeug der eingangs genannten Art ist beispielsweise in der DE 20 2007 012 818 U1 beschrieben. Das aus dieser Druckschrift bekannte Schleifwerkzeug wird im Bereich der Podologie eingesetzt und weist einen kappenförmigen Schleifkörper auf. Der Schleifkörper besteht aus einer metallischen Trägerschicht, auf die eine Schleifschicht aufgebracht ist. Weiterhin gehört zu dem Schleifwerkzeug eine Antriebsvorrichtung zum drehenden Antreiben der Schleifkappe, wobei die Antriebsvorrichtung und die Schleifkappe über einen Spannschaft miteinander verbunden sind. Um den Schleifkörper während des Schleifvorgangs zu kühlen, sind in dem Schleifkörper Kühlbohrungen vorgesehen, über die von innen Kühlluft an den zu behandelnden Bereich strömt.

Als nachteilig wird empfunden, dass die Kühlung, gerade bei längeren Behandlungen, die Schleifwärme nicht vollständig abführen kann, so dass es zu lokalen Hitzeschäden kommen kann. Um dies zu vermeiden ist beispielsweise ein Fußpfleger darauf angewiesen, dass ihm sein Patient mitteilt, wann die Behandlung für ihn hitzebedingt unangenehm wird. Allerdings ist das Risiko, bei der Behandlung Verbrennungen zu erleiden, unter anderem bei Diabetikern besonders hoch, da deren Temperaturempfinden an den Füßen oft gestört ist.

Aus der DE 20 2009 006 069 U1 ist eine Polierscheibe aus einem Polyurethanschaum bekannt. Die weiche Polierscheibe weist thermochrome Farbmittel auf, um einem Benutzer durch Farbumschläge optisch anzuzeigen, dass die Polierscheibe zu heiß wird und infolge dessen die Lackoberfläche schädigen kann. Die thermochromen Farbmittel sind in Mikrokapseln eingeschlossen, die während der Herstellung der Polierscheibe dem Weichschaum beigemischt werden. Die Mikrokapseln enthalten ferner ein niedrigschmelzendes thermoplastisches Harz und eine Aktivatorverbindung. Um während des Poliervorgangs für die Lackoberfläche schädliche Temperaturerhöhungen visuell wahrnehmen zu können, schlägt der thermochrome Farbstoff bei einer definierten Farbumschlagstemperatur um. Das niedrigschmelzende thermoplastische Harz hat im Bereich dieser Farbumschlagstemperatur seinen Schmelzbereich, so dass durch das Aufschmelzen des Harzes das thermochrome Farbmittel mit der Aktivatorverbindung reagieren kann.

Als nachteilig wird empfunden, dass das thermoplastische Harz zunächst schmelzen muss, bevor das thermochrome Farbmittel umschlagen kann. Zudem ist die Herstellung der Polierscheibe aufwendig, da die thermochromen Farbmittel zusammen mit der Aktivatorverbindung und dem thermoplastischen Harz in Mikrokapseln eingebunden werden müssen, um diese anschließend dem Weichschaum beizumischen. Zudem ist nachteilig, dass mit dem Weichschaum keine spanenden oder trennenden Fertigungsschritte an Oberflächen von zu behandelnden Werkstoffen durchgeführt werden können.

Die EP 2 578 108 A1 offenbart ein Dentalwerkzeug mit einem Arbeitsbereich, welcher mit abrasiven Partikeln belegt ist. Die abrasiven Partikel sind zumindest zum Teil in eine galvanisch auf der Oberfläche des Arbeitsbereichs ausgebildete Trägerschicht eingebettet, wobei auf der Trägerschicht zumindest eine, die abrasiven Partikel zumindest teilweise umschließende Deckschicht angeordnet ist. Die Deckschicht kann aus einem farbigen Kunststoffmaterial gefertigt sein, um dem Anwender einen Hinweis auf den Verschleißzustand zu geben.

Ausgehend von diesem Stand der Technik ist es eine Aufgabe der vorliegenden Erfindung, einen Schleifkörper beziehungsweise ein Schleifwerkzeug bereitzustellen, mittels dem hitzebedingte Schäden oder Schmerzen an den zu behandelnden Stellen während eines spanenden Fertigungsverfahrens vermieden werden. Darüber hinaus ist es eine Aufgabe der vorliegenden Erfindung, eine Verwendung eines solchen Schleifkörpers oder eines solchen Schleifwerkzeuges bereitzustellen, mittels dessen hitzebedingte Schäden oder Schmerzen an den zu behandelnden Stellen eines menschlichen Patienten vermieden werden.

Zur Lösung dieser Aufgabe schafft die vorliegende Erfindung ein Schleifwerkzeug gemäß Anspruch 1.

Erfindungsgemäß wird der Effekt der Thermochromie ausgenutzt, wonach bestimmte Substanzen bei Erwärmung beziehungsweise Abkühlung ihre Farbe ändern. Auf diese Weise kann ein Benutzer des Schleifkörpers, beispielsweise ein Podologe oder ein Mechaniker, erkennen, wie heiß insbesondere die Außenfläche des Schleifkörpers ist. Somit kann der Benutzer frühzeitig den Schleifvorgang unterbrechen, um eine Überhitzung der zu behandelnden Stelle zu verhindern. Damit eignet sich der erfindungsgemäße Schleifkörper vor allem für die Behandlung von Patienten, beispielsweise im Bereich der Podologie, der Maniküre oder im Dentalbereich. Darüber hinaus kann der Schleifkörper auch bei Schleifarbeiten an Werkstoffen eingesetzt werden, bei denen eine Überhitzung durch die beim Schleifen entstehende Reibungswärme sicher verhindert werden muss.

Die Schleifschicht ist mehrschichtig aufgebaut und weist zumindest eine innere und eine äußere Bindungsschicht auf. Mit anderen Worten weist die Schleifschicht einen mehrschichtigen Aufbau mit mehreren Bindungsschichten auf, wobei unter Bindungsschichten solche Schichten verstanden werden, die ein Bindemittel aufweisen. Grundsätzlich können neben der inneren und der äußeren Bindungsschicht weitere mittlere Bindungsschichten in der Schleifschicht vorgesehen sein, so dass auch mehr als die zwei genannten Bindungsschichten möglich sind.

In bevorzugter Weise ist vorgesehen, dass der Schleifkörper mehrschichtig aufgebaut ist und eine Trägerschicht aufweist. Die Trägerschicht kann beispielsweise aus einem starren formstabilen Schleifmittelträger aus Metall oder einem anderen formstabilen Material hergestellt sein. Alternativ kann es sich bei der Trägerschicht auch um eine flexible Schleifmittelträgerschicht aus einem Gewebe, insbesondere Baumwolle, handeln. Zum Beispiel kann die Trägerschicht aus Baumwolle mit einer Imprägniermasse ausgerüstet sein, auf welche die Schleifschicht mit dem zumindest einen Bindemittel insbesondere unmittelbar aufgetragen werden kann. Dagegen kann der Schleifkörper auch ohne eine Trägerschicht auskommen. Dies ist insbesondere dann der Fall, wenn der Schleifkörper als längliche Schleifstifte oder scheibenförmige Schleifsteine gestaltet ist.

Vorteilhafterweise umfassen die thermochromen Farbmittel eine Menge thermochromer Farbpigmente mit gleichen Eigenschaften oder zumindest eine Mischung diverser thermochromer Farbpigmente. Alternativ oder zusätzlich können die thermochromen Farbmittel einen thermochromen Farbstoff oder zumindest eine Mischung diverser thermochromer Farbstoffe umfassen. Der thermochrome Farbstoff zeichnet sich dadurch aus, dass er in der Schleifschicht, insbesondere dem zumindest einen Bindemittel, löslich gebunden ist, wogegen die thermochromen Farbpigmente im Allgemeinen unlöslich sind und der Schleifschicht, insbesondere dem zumindest einen Bindemittel, beigemischt werden.

Die innere Bindungsschicht ist als eine Aufbauschicht mit dem zumindest einen Bindemittel und die äußere Bindungsschicht ist als eine Deckschicht mit einem weiteren Bindemittel gestaltet. Grundsätzlich kann für die Aufbauschicht und die Deckschicht auch das gleiche Bindungsmittel verwendet werden. Am Beispiel des zweischichtigen Aufbaus der Schleifschicht wird insbesondere unmittelbar auf eine Trägerschicht eine innere Bindungsschicht, nämlich die Aufbauschicht, aufgebracht. Die Schleifkörner werden auf die Aufbauschicht aufgetragen, wobei diese insbesondere elektrostatisch oder mechanisch gestreut sein können. Das zumindest eine Bindemittel der Aufbauschicht hält die Schleifkörner in der Schleifschicht, um einen Kornausbruch zu vermeiden. Anschließend wird über die Aufbauschicht eine äußere Bindungsschicht, nämlich die Deckschicht aufgetragen. Durch die Deckschicht wird der Halt der Schleifkörner in der Schleifschicht verbessert. Durch den mehrschichtigen Aufbau der Schleifschicht wird somit die Gefahr eines Kornausbruchs deutlich reduziert. Die abschließende Deckschicht bildet keine ebene, glatt abschließende Oberfläche der Schleifschicht aus, sondern bildet aufgrund der teilweise aus der Aufbauschicht vorstehenden Schleifkörper eine zerklüftete, unebene Schleifoberfläche. Mit Beginn eines Schleifvorgangs werden bei einem neuen Schleifkörper zunächst die Bereiche der Deckschicht um die Schleifkörner abgetragen, wodurch die Schleifkörner bereichsweise freigelegt werden. Mit fortschreitendem Gebrauch des Schleifkörpers werden immer größere Bereiche der Deckschicht abgetragen, bis die Deckschicht vollständig verschlissen ist. Durch den mehrschichtigen Aufbau der Schleifschicht werden die Schleifkörner auch nach Abbau der Deckschicht zumindest noch von der Aufbauschicht gehalten. Wenn der Schleifkörper keine Trägerschicht aufweist, wie dies beispielsweise bei länglichen Schleifsteinen oder runden Schleifscheiben der Fall sein kann, weist der Schleifkörper nur die Schleifschicht mit mehreren Bindungsschichten, in denen die Schleifkörner gebunden sind.

Des Weiteren können auf der Deckschicht wenigstens eine weitere Überzugsschicht, beispielsweise eine Antihaftschicht vorgesehen sein, um eine Anhaftung von Schleifstaubpartikeln an einer Außenfläche des Schleifkörpers zu vermeiden. Dadurch wird beim Schleifen das Zusetzen des Schleifkörpers reduziert. Dabei kann die Antihaftschicht nicht nur als äußerste Schicht auf dem Schleifkörper vorgesehen sein, sondern kann ebenso in einer der Bindungsschichten des Schleifkörpers integriert sein.

Gemäß einem Aspekt der vorliegenden Erfindung ist vorgesehen, dass die innere Bindungsschicht frei von thermochromen Farbmitteln ist. Insbesondere sind die thermochromen Farbmittel nur in der Deckschicht vorgesehen. Somit kann der Benutzer bei Verwendung des Schleifkörpers nicht nur dessen Außenflächentemperatur feststellen, sondern erkennt durch den Abrieb der zumindest in vorgegebenen Temperaturbereichen farbigen Deckschicht den Grad des Verschleißes des Schleifkörpers. Weiterhin kann die Deckschicht mit den thermochromen Farbmitteln auch nachträglich auf einem Schleifkörper aufgetragen werden, um herkömmliche Schleifkörper mit thermochromen Farbmitteln auszustatten. Zudem können neben der Deckschicht auch weitere Bindungsschichten, insbesondere mittlere Bindungsschichten zwischen der Aufbauschicht und der Deckschicht, thermochrome Farbmittel umfassen, um insbesondere den Verschleiß einzelner Bindungsschichten schrittweise wahrnehmen zu können.

Um die thermochromen Farbmittel besser von außen erkennen zu können, kann das Bindemittel der Deckschicht ein transparentes Bindemittel sein. Zudem wird die eingenommene Farbe der thermochromen Farbmittel durch das Bindemittel nicht verfälscht und unterhalb der Deckschicht liegende Schichten mit deren gegebenenfalls vorgesehenen Eigenfarben sind besser zu erkennen. Besonders gute Ergebnisse wurden dadurch erreicht, dass das Bindemittel der Deckschicht ein transparenter Duroplast beziehungsweise ein transparentes duroplastisches Bindemittel ist. Somit kann in der Deckschicht oder einer mittleren Bindungsschicht ein möglichst transparentes und farbloses Harz eingesetzt und auf Füllstoffe, mit Ausnahme eventuell beigemischter thermochromer Farbmittel und/oder Schleifkörner, weitestgehend verzichtet werden. Vorzugsweise ist der Harzanteil der Deckschicht abzüglich der Schleifkörner nach dem Aushärten größer als 70 Prozent, insbesondere 90 Prozent und in bevorzugter Weise größer als 95 Prozent. Durch die zumindest im Wesentlichen transparente, transluzent beziehungsweise wenig deckende Deckschicht wird sichergestellt, dass bei einem Verschleiß der Deckschicht deren Deckkraft kontinuierlich nachlässt. Somit können zwischen den thermochromen Farbmitteln der Deckschicht und einer eventuell darunter liegenden eingefärbten Schicht Mischfarben entstehen, so dass ein Anwender des Schleifkörpers einen kontinuierlichen Verschleiß der Schleifschicht bis zum vollständigen Abtrag der Deckschicht erkennen kann.

In bevorzugter Weise kann das Bindemittel der Aufbauschicht ein organisches oder ein anorganisches Bindemittel sein. Beispielsweise kann es ein Harz, das in dem während des Schleifvorgangs vorkommenden Temperaturbereichs nicht schmilzt, oder ein keramisches Bindemittel sein. Bei keramisch gebundenen Schleifmitteln kann das anorganische Bindemittel der Aufbauschicht zwischen den Schleifkörnern beispielsweise Keramik oder Glas sein. Die Deckschicht wird dann in einem zweiten Schritt nach dem Brennen des anorganischen Bindemittels aufgetragen.

Gemäß einem Aspekt der vorliegenden Erfindung sind in der Aufbauschicht nicht thermochrome Farbmittel vorgesehen. Dies schließt allerdings nicht aus, dass in der Aufbauschicht auch thermochrome Farbmittel vorgesehen sein können. Beispielsweise kann das nicht-thermochrome Farbmittel eine rote Eigenfarbe aufweisen. Somit können diverse Farbwechsel realisiert werden, wobei hier ein möglicher Farbwechsel exemplarisch erläutert ist: Die thermochromen Farbmittel sind beispielsweise nur in der Deckschicht vorgesehen und weisen eine Mischung thermochromer Farbmittel auf, die zwei unterschiedliche Gruppen thermochromer Farbpigmente umfasst. Die erste Gruppe weist blaue thermochrome Farbpigmente auf, die bei einer Farbumschlagstemperatur von 40 Grad Celsius transparent werden, und die zweite Gruppe weist gelbe thermochrome Farbpigmente auf, die bei einer Farbumschlagstemperatur von 60 Grad Celsius transparent werden. Die Aufbauschicht ist hier mit einem nicht-thermochromen Farbmittel rot eingefärbt. Somit ergibt sich bei Raumtemperatur eine Mischfarbe, welche die Schleifschicht grün erscheinen lässt. Steigt die Temperatur der Schleifschicht über 40 Grad Celsius, ist die Schleifschicht gelb und über 60 Grad Celsius ist diese rot. Wenn die relative Pigmentmenge der blauen thermochromen Farbpigmente gegenüber den gelben thermochromen Farbpigmenten erhöht wird, könnte der Farbverlauf wie folgt aussehen: Bei Raumtemperatur ist die Schleifschicht blau bis blau/grün. Um die 40 Grad Celsius geht die Farbe der Schleifschicht über von grün nach grün/gelb und oberhalb von 60 Grad Celsius wird diese rot. Neben der Möglichkeit zur Bestimmung der Temperatur der Schleifschicht kann durch die eingefärbte Aufbauschicht der Verschleiß der Deckschicht besser wahrgenommen werden. Während des Gebrauchs des Schleifkörpers wird die Deckschicht lokal abgetragen, bis diese verbraucht ist. Die Schleifkörner nutzen mit der Zeit ab und/oder verstumpfen, so dass der gebrauchte Schleifkörper aufgrund der größeren Schleifoberfläche mehr Reibungswärme als ein neuer Schleifkörper entwickelt. Wenn die Deckschicht abgetragen wird oder vollständig verbraucht ist, steigt das Risiko eines Kornausbruchs der Schleifkörner aus der Aufbauschicht. Durch die Eigenfarbe der Aufbauschicht ist somit der Grad der Abnutzung der mit thermochromen Farbmitteln versehenen Schleifschicht, insbesondere Deckschicht von außen erkennbar.

Weiterhin kann auf einer zur Schleifoberfläche weisenden Oberfläche der Trägerschicht eine nicht-thermochrome Farbe aufgetragen sein. Dadurch wird eine weitere Möglichkeit zur Erkennung des Grades der Abnutzung des Schleifkörpers bereitgestellt, die bei einer transparenten Aufbauschicht gut erkennbar ist.

In bevorzugter Weise ist zumindest eine Teilmenge der Schleifkörner transparent gestaltet. Insbesondere können alle Schleifkörner transparent sein. Als transparente Schleifkörner eignen sich besonders Schleifkörner aus Einkristallkorund. Durch die transparente Ausgestaltung der Schleifkörner sind die thermochromen Farbmittel und eventuell vorgesehene nicht-thermochrome Farbmittel besser zu erkennen und deren Farben werden nicht durch eine Eigenfarbe der Schleifkörner verfälscht.

Zusätzlich oder alternativ zu den transparenten Schleifkörnern kann zumindest eine Teilmenge der Schleifkörner farbig gestaltet sein. Insbesondere können alle Schleifkörner farbig gestaltet sein. Je nach Schleifaufgabe können unterschiedliche Schleifkörner von Vorteil sein, so dass nicht nur oder anstatt der transparenten Einkristallkorunde beispielsweise rotes Edelkorund, grünes Siliziumkarbid oder weiße Schleifkörner, beispielsweise Keramikkorn, Sol-Gel-Korund bzw. Edelkorund weiß, einsetzbar sind. Bei farbigen Schleifkörnern könnte die Farbe der Aufbauschicht durch Mischung des Bindemittels der Aufbauschicht mit nicht-thermochromen Farbmitteln an die Farbe der Schleifkörner angepasst werden.

Um besonders gute Schleifergebnisse mit dem Schleifkörper erzielen zu können, können die Schleifkörner eine Korngröße zwischen 30 und 1400 Mikrometer aufweisen. Durch die großen Korngrößen ist der Spanraum zwischen den Schleifkörner derart groß, dass der Schleifkörper beispielsweise beim Abtragen von Hornhaut an menschlichen Füssen nicht zusetzt. Ist der Spanraum dagegen zu klein, werden das Spanvolumen beziehungsweise die Spanleistung des Schleifkörpers verringert und die Gefahr steigt, dass der Schleifkörper und der Patient beziehungsweise das zu behandelnde Bauteil durch eine schnell ansteigende Außenflächentemperatur des Schleifkörpers Schaden nehmen.

In bevorzugter Weise umfassen die thermochromen Farbmittel reversible und/oder irreversible thermochrome Farbmittel. Am Beispiel eines im kühlen Zustand, beispielsweise bei Raumtemperatur, dunkelfarbigen thermochromen Farbmittel kann dem Benutzer durch Veränderung der Farbe ein Temperaturanstieg signalisiert werden. Die anfänglich dunklen thermochromen Farbmittel können beispielsweise durch einen Farbumschlag ins Rote den Temperaturanstieg anzeigen. Somit kann der Benutzer auf einfache Weise die Außenflächentemperatur des Schleifkörpers bestimmen. Werden beispielsweise reversible Farbmittel verwendet, wird dem Benutzer durch die Farbänderung nicht nur die Erwärmung des Schleifkörpers angezeigt, sondern auch dessen Abkühlung, da der Schleifkörper im abgekühlten Zustand wieder seine ursprüngliche Farbei annimmt. Werden zusätzlich oder alternativ irreversible Farbmittel eingesetzt, kann dem Benutzer dauerhaft angezeigt werden, dass der Schleifkörper beispielsweise oberhalb einer maximal erlaubten Außenflächentemperatur betrieben wurde, indem bei der maximal erlaubten Außenflächentemperatur ein irreversibler Farbumschlag vorgesehen ist. Dadurch wird bleibend angezeigt, dass der Schleifkörper überhitzt wurde und für die weitere Verwendung nicht mehr geeignet ist. Solange der Schleifkörper unterhalb der vorgegebenen maximalen Temperatur betrieben wird, nimmt der Schleifkörper dann im abgekühlten Zustand wieder seine ursprüngliche Farbe an. Schließlich können auch nur irreversible Farbmittel vorgesehen sein, um beispielsweise bereits bei Erreichen einer vorgegebenen Außenflächentemperatur farblich umzuschlagen. Somit kann dauerhaft angezeigt werden, dass der Schleifkörper bereits einmal benutzt wurde. Dies gewährleistet, dass stets nur neue Schleifkörper mit einer einwandfreien Schleifschicht verwendet werden. Am einfachsten wird dies sichergestellt, indem die Farbumschlagstemperatur knapp oberhalb der Raumtemperatur liegt.

Zweckmäßigerweise sind die thermochromen Farbpigmente derart ausgebildet, dass ihre Farbe bei einer Außenflächentemperatur zwischen 20° Celsius und 90° Celsius wenigstens einmal umschlägt. Je nach gewünschter Schleifanwendung können die thermochromen Farbpigmente aber auch derart ausgebildet sein, dass ihre Farbe bei einer Außenflächentemperatur zwischen 30° Celsius und 70° Celsius wenigstens einmal umschlägt. Ganz allgemein gilt bei der Auswahl der Farbpigmente, dass zunächst die vorgesehene Schleifanwendung des Schleifkörpers zu bestimmen ist. Ausgehend von dem interessierenden thermosensitiven Bereich des Patienten oder des Werkstücks sind dann solche thermochromen Farbpigmente auszuwählen, deren Farbe in dem Temperaturbereich oder beim Erreichen der maximal erlaubten Schleiftemperatur wenigstens einmal umschlägt. Da sich der Schleifkörper bei den Schleifvorgängen von außen nach innen erwärmt, können auch bei gleicher Schleifanwendung thermochrome Farbpigmente beziehungsweise thermochrome Farbstoffe unterschiedlicher Farbumschlagtemperaturen eingesetzt werden. Je nachdem wo die thermochromen Farbmittel vorgesehen sind, muss somit die wenigstens eine Farbumschlagstemperatur bei weiter außenliegenden thermochromen Farbmitteln entsprechend niedriger sein als bei weiter innenliegenden.

Bevorzugter Weise sind die thermochromen Farbmittel derart ausgebildet, dass ihre Farbe bei einer Außenflächentemperatur zwischen 40° Celsius und 60° Celsius wenigstens einmal umschlägt. Dieser Bereich eignet sich besonders gut für die Podologie.

Besonders vorteilhaft weisen die thermochromen Farbmittel mehrere definierte Farbumschlagstemperaturen auf. Anhand feiner skalierter Farbänderungen kann der Benutzer die Außenflächentemperatur des Schleifkörpers genauer bestimmen. Durch Mischen diverser thermochromer Farbpigmente und/oder thermochromer Farbstoffe können diverse reversible und/oder irreversible Farbverläufe mit unterschiedlichen Farbumschlagstemperaturen erreicht werden.

Weiterhin können die thermochromen Farbmittel derart ausgebildet sein, dass der wenigstens eine Farbumschlag der thermochromen Farbmittel kontinuierlich oder sprunghaft abläuft. Vorteilhafterweise sind die thermochromen Farbmittel derart ausgebildet, dass der wenigstens eine Farbumschlag der thermochromen Farbmittel kontinuierlich abläuft. Auf diese Weise kann der Benutzer den steigenden Temperaturanstieg des Schleifkörpers gut wahrnehmen und geeignete Gegenmaßnahmen zur Abkühlung des Schleifkörpers, beispielsweise das Absetzen des Schleifkörpers von der zu bearbeitenden Oberfläche, vornehmen. Alternativ kann der wenigstens eine Farbumschlag der thermochromen Farbmittel auch sprunghaft ablaufen. Bei einer Mischung verschiedenster thermochromer Farbmittel können kontinuierliche und sprunghaft ablaufende Farbumschläge vorgesehen sein. Zudem können auch thermochrome Farbmittel vorgesehen sein, die bei einer ersten Farbumschlagstemperatur kontinuierlich und bei einer zweiten Farbumschlagstemperatur sprunghaft ihre Farbe ändern.

Vorteilhafterweise sind die thermochromen Farbmittel derart ausgebildet, dass diese bei zumindest der einen Farbumschlagstemperatur transparent werden. Auf diese Weise werden die darunter liegenden Schichten bei Erreichen dieser Farbumschlagstemperatur sichtbar, so dass beispielsweise eine eingefärbte innere Bindungsschicht oder ein Farbauftrag auf der Trägerschicht von außen erkennbar wird. In bevorzugter Weise sind die thermochromen Farbmittel in der Deckschicht derart ausgebildet, dass sie zumindest bei einer Außenflächentemperatur niedriger als 20° Celsius opak und bei wenigstens einer definierten Außenflächentemperatur höher als 20° Celsius transparent sind. Zweckmäßigerweise ist eine unterhalb der Deckschicht angeordnete innere Bindungsschicht oder die Trägerschicht in einer Warnfarbe eingefärbt. Somit können beispielsweise die thermochromen Farbmittel mit steigender Temperatur einen vorgegebenen Farbverlauf durchlaufen und bei einer definierten Außenflächentemperatur höher als 20° Celsius transparent werden. Dadurch kann eine beispielsweise anfänglich dunkle Farbschicht beim Erwärmen der Außenfläche des Schleifkörpers transparent werden und die unterhalb der Deckschicht angeordnete eingefärbte Schicht durchscheinen lassen. Diese kann zur besseren Wahrnehmbarkeit in einer Warnfarbe, beispielsweise in rot, gelb oder einer auffälligen Neonfarbe gefärbt sein. Ferner kann auch eine Farbe gewählt werden, die der Farbe des Abriebs bei bestimmungsgemäßem Gebrauch entspricht. Somit würde der Schleifkörper sowohl bei vollständiger Bedeckung mit Abrieb als auch bei erhöhter Temperatur ein und dieselbe Farbe annehmen. Der Benutzer erkennt dann, dass er die Arbeiten unterbrechen muss. Sofern der Schleifkörper mit Abrieb zugesetzt ist, ändert sich die Farbe nicht mehr und der Benutzer erkennt, dass er den Schleifkörper nicht weiter verwenden kann, oder die Farbe ändert sich wieder, so dass angezeigt wird, dass der Schleifkörper wieder normal temperiert ist. Ebenso kann die Deckschicht auch derart ausgebildet sein, dass sie zumindest bei einer Außenflächentemperatur niedriger als 20° Celsius transparent und bei wenigstens einer definierten Außenflächentemperatur höher als 20° Celsius opak ist. So kann eine anfängliche unter der Deckschicht durchscheinende Farbe durch die Deckschicht verdeckt werden, um auf diese Weise den Temperaturanstieg anzuzeigen.

Des Weiteren können die thermochromen Farbmittel derart ausgebildet sein, dass ein Farbumschlag bei einer ersten Farbumschlagstemperatur und ein weiterer Farbumschlag bei einer zweiten Farbumschlagstemperatur sind. Vorteilhafterweise ist der Farbumschlag bei der ersten Farbumschlagstemperatur reversibel und der Farbumschlag bei der zweiten Farbumschlagstemperatur irreversibel. Bevorzugter Weise ist die zweite Farbumschlagstemperatur höher ist als die erste Farbumschlagstemperatur. Damit kann der erste Farbumschlag beispielsweise in einem für den zu bearbeitenden Werkstoff oder für den Patienten gefahrlosen Temperaturbereich reversibel sein, so dass der Schleifkörper mit dem Abkühlen wieder seine ursprüngliche Farbe annimmt. Sollte dagegen der Schleifkörper über eine definierte Temperatur erhitzt werden, die beispielsweise für den Werkstoff oder den Patienten schädlich ist, zu Verbrennungen führt oder den Schleifkörper unbrauchbar macht, kann die zweite Farbumschlagstemperatur irreversibel sein. Ebenso kann die zweite Farbumschlagstemperatur kurz über der Raumtemperatur liegen, so dass der Benutzer sofort erkennt, dass der Schleifkörper bereits einmal benutzt wurde. In diesem Fall liegt die erste Farbumschlagstemperatur im Bereich der Raumtemperatur, damit sich die Farbe des Schleifkörpers nicht bereits vor dem Gebrauch irreversibel ändert.

In bevorzugter Weise ist vorgesehen, dass der Schleifkörper als ein rotationssymmetrischer Schleifkopf gestaltet und mit einer Antriebswelle zum drehenden Antreiben verbindbar ist. Beispielsweise ist der Schleifkörper als eine Schleifkappe mit einer Trägerschicht aus einem flexiblen Material, beispielsweise aus Baumwolle hergestellt. Ebenso kann der Schleifkörper als eine Schleifscheibe gestaltet sein.

Des Weiteren ist ein weiterer Gegenstand der vorliegenden Erfindung die Verwendung des vorbeschriebenen Schleifwerkzeuges zur Behandlung von menschlichen Körperstellen. In bevorzugter Weise werden der Schleifkörper beziehungsweise das Schleifwerkzeug für die Fußpflege, also im Rahmen der Podologie, verwendet.

Ein bevorzugtes Ausführungsbeispiel wird im Folgenden anhand der Figuren erläutert. Hierin zeigt:
- Figur 1: einen Längsschnitt einer Ausführungsform eines erfindungsgemäßen Schleifwerkzeugs; und
- Figur 2: einen Längsschnitt einer Teilansicht eines erfindungsgemäßen Schleifkörpers.

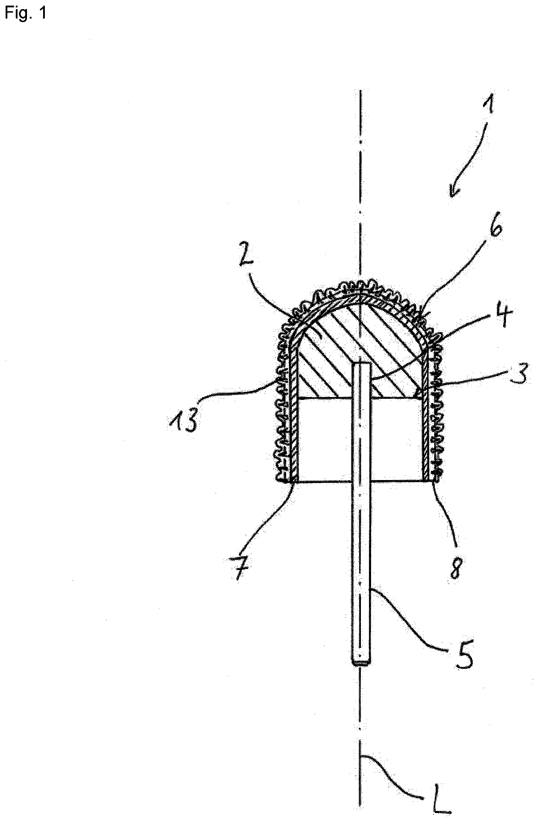

In Figur 1 ist eine Ausführungsform eines erfindungsgemäßen Schleifwerkzeugs zur Fußpflege dargestellt, mit dem insbesondere verhornte Fußpartien geschliffen werden können.

Das Schleifwerkzeug umfasst einen rotationssymmetrischen Schleifkopf 1, der einen aus Harz gegossenen Kern 2 aufweist. Der Kern ist halbkugelförmig ausgebildet und weist an einem abgeflachten Ende 3 eine zentrale Bohrung 4 auf. In diese Bohrung 4 ist ein länglicher Schaft 5 gepresst, der vorzugsweise aus Metall hergestellt ist. Weiterhin ist eine nicht dargestellte Antriebsvorrichtung vorgesehen, um den Schleifkopf 1 um seine Längsachse L drehend anzutreiben. Auf dem Kern ist vollflächig eine Schleifkappe 6 aufgeklebt, wobei diese den Kern 2 an dessen abgeflachtem Ende 3 in Längsrichtung überragt.

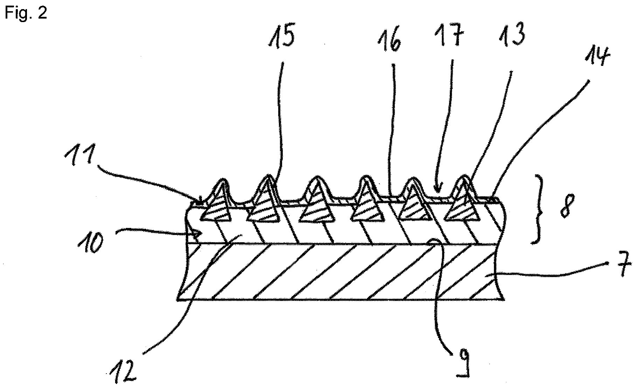

In Figur 2 ist ein Längsschnitt der erfindungsgemäßen Schleifkappe 6 gezeigt. Erkennbar ist, dass die Schleifkappe 6 mehrschichtig aufgebaut ist und eine Trägerschicht 7 und eine Schleifschicht 8 aufweist. Die Trägerschicht 7 ist aus einem flexiblen Baumwollgewebe hergestellt und ist mit dem in Figur 2 nicht dargestellten Kern 2 verklebt. Auf einer von dem Kern 2 wegweisenden Außenseite 9 der Trägerschicht 7 ist die Schleifschicht 8 aufgetragen.

Die Schleifschicht 8 ist mehrschichtig ausgebildet und weist eine innere und eine äußere Bindungsschicht, nämlich eine Aufbauschicht 10 und eine Deckschicht 11 auf. Die Aufbauschicht 10 ist auf der Außenseite 9 der Trägerschicht 7 aufgebracht. Diese weist ein keramisches Bindemittel 12 auf, das in einer roten nicht-thermochromen Farbe eingefärbt ist. In dem keramischen Bindemittel 12 der Aufbauschicht 10 sind Schleifkörner 13 gebunden. Die Schleifkörner 13 sind transparente Einkristallkorunde, die vorzugsweise elektrostatisch in der Aufbauschicht 10 gestreut sind. Diese weisen eine Korngröße von etwa 425 Mikrometer auf. Erkennbar ist, dass die Aufbauschicht 10 und die teilweise vorstehenden Schleifkörner 13 eine unebene, zerklüftete Oberfläche 14 bilden. Auf diese zerklüftete Oberfläche 14 ist die Deckschicht 11 aufgetragen, welche die Aufbauschicht 10 und die Schleifkörner 13 mit einer verhältnismäßig dünnen Schicht überzieht und die Schleifkörner 13 zusätzlich bindet. Die Deckschicht 11 weist ein transparentes duroplastisches Bindemittel 15 auf, das thermisch aushärtbar ist. Entsprechend der zerklüfteten Oberfläche 14 weist auch die Deckschicht 11 eine unebene Außenfläche 16 auf. Zwischen den einzelnen Schleifkörnern 13 sind Spanräume 17 definiert, die zur Aufnahme abgetrennter Hornhautspäne während des Schleifens dienen.

Zur Bestimmung der Außenflächentemperatur der Schleifkappe 6 sind im transparenten Bindemittel 15 der Deckschicht 11 thermochrome Farbmittel gebunden. Die thermochromen Farbmittel weisen eine Mischung reversibler Farbpigmente auf, die zwei gleichverteilte Gruppen umfasst. Die erste Gruppe weist blaue thermochrome Farbpigmente auf, die bei einer Farbumschlagstemperatur von 40 Grad Celsius transparent werden, und die zweite Gruppe weist gelbe thermochrome Farbpigmente auf, die bei einer Farbumschlagstemperatur von 60 Grad Celsius transparent werden. Somit ist die mit den thermochromen Farbpigmenten versehene Deckschicht 11 bei Raumtemperatur in einem grünen Farbton eingefärbt.

Im Betrieb entnimmt der Podologe den Schleifkopf 1 aus einer sterilen Verpackung und spannt diese mittels des Schafts 5 in ein Spannfutter der Antriebsvorrichtung ein. Während des Schleifvorgangs drückt der Podologe den Schleifkopf 1 beispielsweise gegen einen verhornten Fußbereich des Patienten.

Durch die Reibung zwischen der Schleifschicht 8 und dem Fuß des Patienten entsteht Reibungswärme, die zu einem Anstieg der Außenflächentemperatur der Schleifkappe 6 führt. Die im kalten Zustand bei Raumtemperatur noch grüne Deckschicht 11 wird ab einer Außenflächentemperatur der Schleifkappte 6 von 40 Grad Celsius gelb. Somit erkennt der Podologe, dass die Außenflächentemperatur der Schleifkappe 6 in einer für den Patienten angenehmen Temperatur oberhalb von 40 Grad Celsius, aber unterhalb von 60 Grad Celsius liegt.

Wenn der Podologe den Schleifkörper 1 nicht absetzt, steigt die Außenflächentemperatur der Schleifkappe 6 mit längerer Schleifdauer weiter an. Überschreitet die Außenflächentemperatur 60 Grad Celsius, wird die Deckschicht 11 transparent und die rote Aufbauschicht 10 ist zu erkennen. Dadurch wird dem Podologen signalisiert, dass die Außenflächentemperatur eine für den Patienten unangenehme Temperatur erreicht hat und der Schleifvorgang zur Abkühlung der Schleifkappe 6 unterbrochen werden sollte.

Sobald der Podologe den Schleifkörper 1 vom Fuß abhebt, kann die Schleifkappe 6 abkühlen. Dann liegen auch die Spanräume 17 der Schleifschicht 8 frei, in welche die von der Schleifschicht 8 abgehobenen Späne der Hornhaut während des Schleifvorgangs transportiert wurden. Die Späne fallen somit aus den Spanräumen 14 hinaus beziehungsweise können ausgeblasen werden.

Darüber hinaus eignen sich die thermochromen Farbpigmente in der Deckschicht 11 nicht nur zur Anzeige der Außenflächentemperatur der Schleifschicht 8, sondern auch zur Bestimmung des Verschleißes der Schleifschicht 8. Denn mit Beginn des ersten Schleifvorgangs wird die Schleifschicht 8 langsam abgetragen. Der Podologe erkennt deren Verschleiß dadurch, dass zunächst im Bereich der transparenten Schleifkörner 13 die durch die thermochromen Farbpigmente eingefärbte Deckschicht 11 abgetragen wird und die darunter liegende rote Aufbauschicht 10 stellenweise sichtbar wird. Mit zunehmender Abnutzung der Schleifschicht 8 werden immer größere Flächen der rot eingefärbten Aufbauschicht 10 sichtbar, da die Deckschicht 11 zumindest lokal um die Schleifkörner 13 weiter abgetragen und bei weiterer Benutzung vollständig aufgebraucht wird. Auf diese Weise wird dem Podologen angezeigt, dass die Deckschicht 11, die neben der Aufbauschicht 10 den Schleifkörnern 13 einen zusätzlichen Halt gibt, allmählich verbraucht wird. Um einen Kornausbruch der Schleifkörner 13 zu vermeiden, sollte die Schleifkappe 6 nicht mehr verwendet werden, wenn die Aufbauschicht 10 größtenteils freigelegt ist.

Durch das Mischen diverser thermochromer Farbmittel in den einzelnen Bindungsschichten 10, 11 können verschiedenste Farbverläufe realisiert werden. Zudem können auch irreversible thermochrome Farbmittel vorgesehen werden, um dem Podologen beim Überschreiten einer definierten Außenflächentemperatur der Schleifkappe 6 dauerhaft anzuzeigen, dass diese benutzt wurde und aus hygienischen Gründen nicht weiter verwendet werden sollte.

### Bezugszeichenliste

- 1: Schleifkopf
- 2: Kern
- 3: Ende
- 4: Bohrung
- 5: Schaft
- 6: Schleifkappe
- 7: Trägerschicht
- 8: Schleifschicht
- 9: Außenseite
- 10: Aufbauschicht
- 11: Deckschicht
- 12: Bindemittel der Aufbauschicht
- 13: Schleifkörner
- 14: Oberfläche
- 15: Bindemittel der Deckschicht
- 16: Außenfläche
- 17: Spanräume
- L: Längsachse

## Patentansprüche

1. Schleifwerkzeug (1) mit einem Schleifkörper (6) und Mitteln (5) zum Verbinden des Schleifwerkzeuges (1) mit einer Antriebsvorrichtung zum drehenden Antreiben des Schleifkörpers (6),
wobei der Schleifkörper (6) eine mehrschichtig aufgebaute Schleifschicht (8) mit zumindest einer inneren Bindungsschicht (10), einer äußeren Bindungsschicht (11) und Schleifkörnern (13) aufweist,
wobei die innere Bindungsschicht (10) als eine Aufbauschicht mit zumindest einem Bindemittel (12) und die äußere Bindungsschicht (11) als eine Deckschicht mit einem weiteren Bindemittel (15) gestaltet ist,
**dadurch gekennzeichnet,**
**dass** in der äußeren Bindungsschicht (11) thermochrome Farbmittel vorgesehen sind.

2. Schleifwerkzeug (1) nach Anspruch 1,
**dadurch gekennzeichnet,**
**dass** der Schleifkörper (6) mehrschichtig aufgebaut ist und eine Trägerschicht (7) aufweist.

3. Schleifwerkzeug (1) nach einem der vorhergehenden Ansprüche,
**dadurch gekennzeichnet,**
**dass** die innere Bindungsschicht (10) frei von thermochromen Farbmitteln ist.

4. Schleifwerkzeug (1) nach einem der vorherigen Ansprüche,
**dadurch gekennzeichnet,**
**dass** das Bindemittel (15) der Deckschicht (11) ein transparentes Bindemittel ist.

5. Schleifwerkzeug (1) nach Anspruch 4,
**dadurch gekennzeichnet,**
**dass** das Bindemittel (15) der Deckschicht (11) ein transparenter Duroplast ist.

6. Schleifwerkzeug (1) nach einem der vorherigen Ansprüche,
**dadurch gekennzeichnet,**
**dass** in der Aufbauschicht (10) nicht-thermochrome Farbmittel vorgesehen sind.

7. Schleifwerkzeug (1) nach einem der Ansprüche 2 bis 6,
**dadurch gekennzeichnet,**
**dass** auf einer zur Schleifschicht (8) weisenden Oberfläche (9) der Trägerschicht (7) eine nicht-thermochrome Farbe aufgetragen ist.

8. Schleifwerkzeug (1) nach einem der vorherigen Ansprüche,
**dadurch gekennzeichnet,**
**dass** zumindest eine Teilmenge der Schleifkörner (13) transparent gestaltet ist.

9. Schleifwerkzeug (1) nach einem der vorherigen Ansprüche,
**dadurch gekennzeichnet,**
**dass** zumindest eine Teilmenge der Schleifkörner (13) farbig gestaltet ist.

10. Schleifwerkzeug (1) nach einem der vorherigen Ansprüche,
**dadurch gekennzeichnet,**
**dass** die Schleifkörner (13) eine Korngröße zwischen 30 und 1400 Mikrometer aufweisen.

11. Schleifwerkzeug (1) nach einem der vorherigen Ansprüche,
**dadurch gekennzeichnet,**
**dass** die thermochromen Farbmittel zumindest eine Farbumschlagstemperatur derart aufweisen, dass deren Farbe zwischen 40 Grad Celsius und 60 Grad Celsius wenigstens einmal umschlägt.

12. Schleifwerkzeug (1) nach einem der vorherigen Ansprüche,
**dadurch gekennzeichnet,**
**dass** die thermochromen Farbmittel mehrere definierte Farbumschlagstemperaturen aufweisen.

13. Schleifwerkzeug (1) nach einem der vorherigen Ansprüche,
**dadurch gekennzeichnet,**
**dass** die thermochromen Farbmittel derart ausgebildet sind, dass ein Farbumschlag bei einer ersten Farbumschlagstemperatur und ein weiterer Farbumschlag bei einer zweiten Farbumschlagstemperatur sind, wobei der Farbumschlag bei der ersten Farbumschlagstemperatur reversibel und der Farbumschlag bei der zweiten Farbumschlagstemperatur irreversibel ist, wobei die zweite Farbumschlagstemperatur höher ist als die erste Farbumschlagstemperatur.

14. Verwendung eines Schleifwerkzeugs (1) nach einem der vorherigen Ansprüche zur Behandlung von menschlichen Körperstellen.

15. Verwendung des Schleifwerkzeuges (1) nach Anspruch 14,
**dadurch gekennzeichnet,**
**dass** das Schleifwerkzeug (1) für die Fußpflege verwendet wird.

## Claims

1. Grinding tool (1) with an abrasive body (6) and elements (5) for connecting the grinding tool (1) to a driving device for rotatingly driving the abrasive body (6),
wherein the abrasive body (6) has an abrasive layer (8) built up from a plurality of layers, with at least one inner bonding layer (10), one outer bonding layer (11) and abrasive grains (13),
wherein the inner bonding layer (10) is formed as a base layer with at least one binding agent (12) and wherein the outer bonding layer (11) is formed as a cover layer with a further binding agent (15), **characterized in**
**that** the outer bonding layer (11) is provided with thermochromic colouring agents.

2. Grinding tool (1) according to claim 1,
**characterised in**
**that** the abrasive body (6) is built up from a plurality of layers and has a substrate layer (7).

3. Grinding tool (1) according to one of the preceding claims,
**characterised in**
**that** the inner bonding layer (10) is free of thermochromic colouring agents.

4. Grinding tool (1) according to one of the preceding claims,
**characterised in**
**that** the binding agent (15) of the cover layer (11) is a transparent binding agent.

5. Grinding tool (1) according to claim 4,
**characterised in**
**that** the binding agent (15) of the cover layer (11) is a transparent thermosetting plastic.

6. Grinding tool (1) according to one of the preceding claims,
**characterised in**
**that** non-thermochromic colouring agents are provided in the base layer (10).

7. Grinding tool (1) according to one of claims 2 to 6,
**characterised in**
**that** a non-thermochromic colour is applied on an upper face (9) of the substrate layer (7) facing to the abrasive layer (8).

8. Grinding tool (1) according to one of the preceding claims,
**characterised in**
**that** at least a partial amount of the abrasive grains (13) is formed transparent.

9. Grinding tool (1) according to one of the preceding claims,
**characterised in**
**that** at least a partial amount of the abrasive grains (13) is formed coloured.

10. Grinding tool (1) according to one of the preceding claims,
**characterised in**
**that** the abrasive grains (13) have a grain size between 30 and 1400 micrometers.

11. Grinding tool (1) according to one of the preceding claims,
**characterised in**
**that** the thermochromic colouring agents have at least a colour change temperature such, that their colour changes at least once between 40 degree Celsius and 60 degree Celsius.

12. Grinding tool (1) according to one of the preceding claims,
**characterised in**
**that** the thermochromic colouring agents have several defined colour change temperatures.

13. Grinding tool (1) according to one of the preceding claims,
**characterised in**
**that** the thermochromic colouring agents are formed such, that a colour change takes place at a first colour change temperature and a further colour change takes place at a second colour change temperature, wherein the colour change at the first colour change temperature is reversible and the colour change at the second colour change temperature is irreversible, wherein the second colour change temperature is higher than the first colour change temperature.

14. Use of a grinding tool (1) according to one of the preceding claims,
for treating human body parts.

15. Use of a grinding tool (1) according to claim 14,
**characterised in**
**that** the grinding tool (1) is used for podiatry.

## Revendications

1. Outil abrasif (1) comprenant un corps abrasif (6) et des moyens (5) pour relier l'outil abrasif (1) à un dispositif d'entraînement pour l'entraînement en rotation du corps abrasif (6),
dans lequel le corps abrasif (6) présente une couche abrasive (8) multicouches comprenant au moins une couche de liaison intérieure (10), une couche de liaison extérieure (11) et des grains abrasifs (13), dans lequel la couche de liaison intérieure (10) est conçue en tant que couche de montage avec au moins un liant (12) et la couche de liaison extérieure (11) est conçue en tant que couche de couverture avec un liant supplémentaire (15),
**caractérisé en ce que**
des colorants thermochromes sont prévus dans la couche de liaison extérieure (11).

2. Outil abrasif (1) selon la revendication 1, **caractérisé en ce que** le corps abrasif (6) est multicouches et présente une couche porteuse (7).

3. Outil abrasif (1) selon la revendication 1 ou 2, **caractérisé en ce que** la couche de liaison intérieure (10) est exempte de colorants thermochromes.

4. Outil abrasif (1) selon l'une des revendications précédentes, **caractérisé en ce que** le liant (15) de la couche de couverture (11) est un liant transparent.

5. Outil abrasif (1) selon la revendication 4, **caractérisé en ce que** le liant (15) de la couche de couverture (11) est une résine thermodurcissable transparente.

6. Outil abrasif (1) selon l'une des revendications précédentes, **caractérisé en ce que** des colorants non thermochromes sont prévus dans la couche de montage (10).

7. Outil abrasif (1) selon l'une des revendications 2 à 6, **caractérisé en ce qu'**un colorant non thermochrome est prévu sur une surface (9) de la couche porteuse (7) orientée vers la couche abrasive (8).

8. Outil abrasif (1) selon l'une des revendications précédentes, **caractérisé en ce qu'**au moins une quantité partielle des grains abrasifs (13) est transparente.

9. Outil abrasif (1) selon l'une des revendications précédentes, **caractérisé en ce qu'**au moins une quantité partielle des grains abrasifs (13) est colorée.

10. Outil abrasif (1) selon l'une des revendications précédentes, **caractérisé en ce que** les grains abrasifs (13) présentent une taille de grain située entre 30 et 1400 micromètres.

11. Outil abrasif (1) selon l'une des revendications précédentes, **caractérisé en ce que** les colorants thermochromes présentent au moins une température de virage de couleur de telle sorte que leur couleur vire au moins une fois entre 40 degrés Celsius et 60 degrés Celsius.

12. Outil abrasif (1) selon l'une des revendications précédentes, **caractérisé en ce que** les colorants thermochromes présentent plusieurs températures de virage de couleur définies.

13. Outil abrasif (1) selon l'une des revendications précédentes, **caractérisé en ce que** les colorants thermochromes sont ainsi conçus qu'un virage de couleur se fait à une première température de virage de couleur et un virage de couleur supplémentaire se fait à une seconde température de virage de couleur, dans lequel le virage de couleur à la première température de virage de couleur est réversible et le virage de couleur à la deuxième température de virage de couleur est irréversible, dans lequel la deuxième température de virage de couleur est supérieure à la première température de virage de couleur.

14. Emploi d'un outil abrasif (1) selon l'une des revendications précédentes pour traiter des endroits du corps humain.

15. Emploi de l'outil abrasif (1) selon la revendication 14, **caractérisé en ce que** l'outil abrasif (1) est employé en pédicure.
